(19) Europäisches Patentamt
European Patent Office
Office européen des brevets

(11) **EP 1 173 546 B1**

(12) **EUROPEAN PATENT SPECIFICATION**

(45) Date of publication and mention
of the grant of the patent:
**09.06.2010 Bulletin 2010/23**

(51) Int Cl.:
*C12N 1/21* *(2006.01)*     *C12N 15/75* *(2006.01)*
*C12P 21/00* *(2006.01)*     *C12P 21/02* *(2006.01)*

(21) Application number: **00912408.2**

(22) Date of filing: **23.03.2000**

(86) International application number:
**PCT/DK2000/000139**

(87) International publication number:
**WO 2000/063346 (26.10.2000 Gazette 2000/43)**

(54) **A BACILLUS PROTEIN PRODUCTION CELL**

BAZILLUS ZELLE FÜR DIE HERSTELLUNG VON PROTEINEN

CELLULE BACILLUS, PRODUCTRICE D'UNE PROTEINE

(84) Designated Contracting States:
**AT BE CH CY DE DK ES FI FR GB GR IE IT LI LU MC NL PT SE**

(30) Priority: **16.04.1999 DK 50699**

(43) Date of publication of application:
**23.01.2002 Bulletin 2002/04**

(73) Proprietor: **Novozymes A/S**
**2880 Bagsvaerd (DK)**

(72) Inventors:
• **JOERGENSEN, Steen, Troels**
**DK-3450 Alleroed (DK)**
• **CHRISTENSEN, Christina, Lund**
**DK-3500 Vaerloese (DK)**

• **KRISTENSEN, Tina**
**DK-2650 Hvidovre (DK)**

(74) Representative: **Lindum, Peter Würtz**
**Novozymes A/S**
**Patents**
**Krogshøjvej 36**
**2880 Bagsværd (DK)**

(56) References cited:
• **F. KUNST ET AL.: 'The complete genome sequence of the gram-positive bacterium bacillus subtilis' NATURE vol. 390, November 1997, pages 249 - 256, XP002080813**
• **DATABASE SWISS PROT [Online] ACCESSION NO. P32689 XP002949852 Retrieved from EMBL**

**Description**

**FIELD OF INVENTION:**

The present invention relates to a *Bacillus* cell for improved production of a polypeptide of interest and a process for producing a polypeptide of interest.

**BACKGROUND OF THE INVENTION:**

Bacillus cells have been widely used for industrial production of polypeptides of interest. Kunst et al., Nature 390: 249-256 (1997) describes the complete genome sequence of the Gram-positive bacterium *Bacillus subtilis.*

**SUMMARY OF INVENTION**

The problem to be solved by the present invention is to provide a *Bacillus* cell capable of producing increased yields of a polypeptide of interest.
The solution is based on that the present inventors have identified that a *Bacillus* cell expressing less than wild-type level of a gene comprising the DNA sequence shown in SEQ ID NO 1 produces increased yields of a polypeptide of interest.
The gene is identified in a *Bacillus subtilis* cell and is termed *yjbH* in said *Bacillus subtilis* cell. See Kunst et al., Nature 390:249-256 (1997).
Further, the inventors have identified a homologous *yjbH* gene in a *B. licheniformis* cell (SEQ ID NO 3).
As stated above (see Background; Kunst et al., Nature 390: 249-256 (1997)) the *yjbH* gene was at the priority dace of the present invention NOT annotated, *i.e.* no known function had been associated with the gene.
Based on the teaching provided herein it is within the skilled persons general knowledge to identify a homologous gene in another *Bacillus* cell, e.g. by DNA sequence homology to the *yjbH* genes disclosed herein (SEQ ID NO 1 and 3) and thereby readily be able to produce a *Bacillus* cell capable of producing an increased yield of a polypeptide of interest according to the solution outlined above.
Further, the DNA sequence shown in SEQ ID NO 1 has very low identity to any other known DNA sequences from any cell. A homology search performed in the publicly known databases such as EMBL, showed that no other sequence had any close identity to the DNA sequence shown in SEQ ID NO 1.
Likewise a homology search in the publicly available SWIS-SPROT database, using the polypeptide sequence shown in SEQ ID NO 2, showed no other polypeptide with any close identity.
Accordingly, in a first aspect the present invention relates to a *Bacillus* cell for improved production of a polypeptide of interest, wherein a gene is inactivated which comprises:

(a) the DNA sequence shown in positions 1 to 828 in SEQ ID NO 1;
(b) a DNA sequence which is at least 70% identical to the DNA sequence of item (a);
(c) a DNA sequence which encodes a polypeptide sequence shown in positions 1 to 275 in SEQ ID NO 2; or
(d) a DNA sequence which encodes a polypeptide sequence which is at least 70% identical to the polypeptide sequence shown in positions 1 to 275 in SEQ ID NO 2.

Further, the present inventors have identified that a partial sequence shown as DNA sequence from position 1 to 147 in SEQ ID NO 1 is highly conserved.
As it is clear from the above a *Bacillus* cell as described herein is highly suitable for the production of a polypeptide of interest.
Accordingly, a second aspect of the present invention is a process for producing a polypeptide of interest comprising following steps::

(i) cultivating a *Bacillus* of the first or second preceding aspects under conditions permitting production of the polypeptide of interest;
(ii) isolating the polypeptide of interest.

**Definitions:**

Prior to a discussion of the detailed embodiments of the invention is provided a definition of specific terms related to the main aspects of the invention,
**[0016]** The term "a gene" denotes herein a gene (a DNA sequence) which is capable of being expressed into a polypeptide within said cell. Accordingly, said gene will be defined as an open reading frame starting from a start codon (normally "ATG", "GTG", or "TTG") and ending at a stop codon (normally "TAA", TAG" or "TGA").

In order to express said gene there must be elements, as known in the art, in connection with the gene, necessary for expression of the gene within the cell. Such standard elements may include a promoter, a ribosomal binding site, a termination sequence, and may be other elements as known in the art.

The term "the *Bacillus* cell expresses less than wild-type levels of a gene" according to the first and second aspects of the invention denotes any alterations of the wild-type cell giving rise to a cell which expresses less than wild-type levels of a gene. These alterations may be alterations of a prompter and/or an open reading frame such as deletions, insertions, frameshifts or any manipulations of the DNA as known in the art.

The expression level of a gene in a *Bacillus* cell altered according to the above is preferably determined by comparing production levels of the polypeptide of interest in said cell with the production level of the polypeptide of interest in the parent non-altered *Bacillus* cell. If the altered cell produces more of the polypeptide of interest when compared to the non-altered cell then the. *Bacillus* cell, according to the first and second aspects of the invention, expresses less than wild-type levels of a gene. The actual assay for the determination of production levels of the polypeptide of interest will depend on the specific polypeptide of interest. It is within the skilled persons general knowledge to choose the appropriate assay.

Identity of DNA sequences.

The DNA sequence identity in relation to the terms "a DNA sequence which is at least 70% identical to the DNA sequence shown in positions 1-828 of SEQ ID NO 1" of the first aspect is determined as the degree of identity between two sequences indicating a derivation of the first sequence from the second. The identity may suitably be determined by means of computer programs known in the art, such as GAP provided in the GCG program package (Program Manual for the Wisconsin Package, Version 8, August 1994, Genetics Computer Group, 575 Science Drive, Madison, Wisconsin, USA 53711)(Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3, the analogous DNA sequences preferred to above exhibits a degree of identity preferably of at least 70%, more preferably at least 75%, more preferably at least 80%, more preferably at least 90%, more preferably at least 95%, more preferably at least 97% with the DNA sequence shown in positions 1-828 of the first aspect of the invention.

Identity of polypentide sequences:

The polypeptide sequence identity in relation to the terms "a DNA sequence which encodes a polypeptide sequence which is at least 70% identical to the polypeptide sequence shown in positions 1 to 275 in SEQ ID NO 2" of the first aspect of the invention is determined as the degree of identity between two sequences indicating a derivation of the first sequence from the second, The homology may suitably be determined by means of computer programs known in the art such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, 443-453). Using GAP with the following settings for polypeptide sequence comparison: GAP creation penalty of 3.0 and GAP extension penalty of 0.1, the polypeptide encoded by an analogous DNA sequence of the invention exhibits a degree of identity preferably of at least 70%, more preferably at least 75%, more preferably at least 80%, more preferably at least 90%, more preferably at least 95%, and especially at least 97% with the polypeptide sequence shown in positions 1 to 275 of SEQ ID NO 2 of the first aspect.

**DETAILED DESCRIPTION OF THE INVENTION:**

**A *Bacillus* cell for improved production of a polypeptide according to the first aspect of the invention:**

The gene as described herein is preferably situated between the two well-known genetic markers in *Bacillus, srfAA* and *thyA*.

The term "situated between the two well known genetic markers in *Bacillus*, *srfAA* and *thyA*" denotes herein that said gene is found between the 3'-end of *srfAA* which is situated at approx. 32° on the *B. subtilis* chromosome, and the 5'-end of *thyA* which is situated at approx. 162.5° on the *B. subtilis* chromosome (F.Kunst et al., 1997, Nature 390 (20): 249-256).

The genetic marker *srfAA* encodes surfactin synthase subunit I in *Bacillus*, reference is made to (Fuma, S. et al., Nucleic Acids Res. 1993; 21(1): 93-7) for further details.

The genetic marker *thyA* encodes thymidylate synthases A in *Bacillus*, reference is made to (Tam, N.H. et al.,

Mol Gen Genet. 1998; 258(4): 427-30) for further details.

The *srfAA* marker is a member of a superfamily of homologous markers that are found in numerous different prokaryotic cells, such as *swrA* in *Serratia liquefaciens* (Lindum et al., J Bacteriol. 1998; 180(23): 6384-8) and *grsA* in *Bacillus brevis* (Krause, M. et al., J Bacteriol. 1988; 170(10): 4669-74).

The *thyA* marker is well known in numerous different prokaryotic cells, such as *Neisseria gonorrhoeae* (Carlson, J.H. et al., FEMS Microbiol Lett. 1997; 151(2): 225-30), *Bacillus amyloliquefaciens* (Tam, N.H. et al., Mol Gen Genet. 1998; 258(4): 427-30), *Mycobacterium tuberculosis* (Cole, S.T. et al., Nature. 1998; 393(6685): 537-44).

The gene as described herein is more preferably situated between the two well-known genetic markers in *Bacillus, mecA* and *tenA* (F.Kunst et al., 1997, Nature 390(20):249-256).

The term "situated between the two well-known genetic markers in *Bacillus, mecA* and *tenA*" denotes herein that said gene is found between the 3'-end of *mecA* which is situated at approx. 105° on the *B. subtilis* chromosome, and the 5'-end of tenA which is situated at approx. 106° on the *B. subtilis* chromosome (F.Kunst et al., 1997, Nature 390(20):249-256).

The genetic marker *mecA* encodes a negative regulator of genetic competence in *Bacillus*, reference is made to (Kong, L. et al., Mol Microbiol 1993 Jul; 9(2): 365-73) for further details.

The genetic marker tenA encodes a polypeptide which regulates production of extracellular enzymes in *Bacillus*, reference is made to (Pang, AS. et al., J. Bacteriol 1991 Jan;173(1):46-54) for further details.

These two markers are well known in numerous different prokaryotic cells, such as *Bacillus firmus* (Guo, D. et al., Bio-chim Biophys Acta 1998 Jan 5; 1389(1): 34-42), *Staphylococcus aureus and S. epidermis* (Ryffel, et al., Gene. 1990 Sep 28; 94(1): 137-8), *Helicobacter pylori* (Tomb, JF. et al., Nature 1997 Aug 7;388(6642):539-47).

Among other based on above-mentioned references it is among the skilled person's general knowledge to identify these genetic marker genes in a particular *Bacillus* cell of interest, e.g. a *Bacillus lentus, Bacillus alkalophilus, Bacillus clausii, Bacillus circulans, Bacillus firmus,* or a *Bacillus thuringiensis* cell.

Preferably, it is a *Bacillus licheniformis, Bacillus subtilis*, or a *Bacillus amyloliquefaciens* cell.

A preferred embodiment of the invention relates to a *Bacillus* cell as described herein; wherein the *Bacillus* cell expresses less than wild-type levels of a gene as described herein due to that said gene is inactivated.

The gene may be inactivated according to any of the strategies well known to the skilled person, such as deletions, insertions of frameshift mutations within the gene or in the promoter.

A further embodiment relates to a *Bacillus* cell as described herein, wherein the polypeptide of interest is an enzyme, such as a protease; a cellulase; a lipase; a xylanase; a phospholipase; or preferably an amylase.

In SEQ ID NO 3 and SEQ ID NO 4 is respectively shown the partial DNA and polypeptide sequences of a *yjbH* gene from *Bacillus licheniformis*. The DNA sequences shown in positions 1 to 147 in SEQ ID NO 1 and the DNA sequence shown in positions 1 to 147 in SEQ ID NO 3 are 76% identical. The polypeptide sequences shown in positions 1 to 49 in SEQ ID NO 2 and the polypeptide sequence shown in positions: 1 to 49 in SEQ ID NO 4 are 79% identical.

All embodiments relating to the first aspect of the invention, e.g. preferred homology identities and the situation of the gene between preferred markers are also preferred embodiments in relation to the aspects mentioned immediately above.

**A process for producing a polypeptide of interest according to the invention:**

An essential element in this process is the use of a *Bacillus* cell as described herein.

The specific cultivation strategy conditions permitting production of the polypeptide of interest may be any of the numerous cultivation protocols known to the skilled person.

Similarly, the specific strategy for isolating the polypeptide of interest of item ii) of the third aspect may be may be any of the numerous isolation protocols known to the skilled person.

**EXAMPLES:**

**MATERIALS AND METHODS**

If not otherwise mentioned, *in vitro* DNA work, transformation of bacterial cells etc. were performed using standard methods of molecular biology (Maniatis, T., Pritsch, E. F., Sambrook, J. "Molecular Cloning. A laboratory manual". Cold Spring Harbor Laboratories, 1982; Ausubel, F. M., et al. (eds.) "Current Protocols in Molecular Biology". John Wiley and Sons, 1995; Harwood, C. R., and Cutting, S. M. (eds.) "Molecular Biological Methods for Bacillus". John Wiley and Sons, 1990).

If not otherwise mentioned enzymes for DNA manipulations were used according to the specifications of the suppliers.

Media used (TY, BPX and LB agar) have been described in EP 0 506 780. LBPSG agar is LB agar supplemented with phosphate (0.01 M K$_3$PO$_4$), glucose (0.4 %), and starch (0.5 %). Agar plates containing dyed amylopectin, as described in WO 94/19454, were used in plate screening for increased amylase production. Minimal medium used was Spizizen's minimal medium in which glucose was replaced by ribose (Spizizen, 1958. Proc. Natl. Acad. Sci. USA 44, 1072-1078).

**α-Amylase assay.**

α-Amylase activity in culture supernatants was measured using the Phadebas® Amylase Test Kit (Pharmacia Diagnosics). The protocol was provided with the Phadebas assay kit.

**Example 1:**

**Inactivation of a gene which when inactivated gives a *Bacillus* subtilis cell capability of increased production of an α-amylase:**

A *Bacillus subtilis* strain expressing a chimeric α-amylase from a chromosomally integrated gene was mutagenised using the mini-Tn*10* transposon pIC333 (Steinmetz, M and Richter, R. 1994, J. Bacteriol. 172: 5019). The strain used was DN1885 *xy1R*::pCJ203, expressing a chimeric α-amylase denoted AmyLQS55-4. The exact composition of this chimeric α-amylase gene and construction of this strain has been published (Christina Lund Jensen (1997) Secretion of chimeric α-amylases from *Bacillus subtilis*. Ph.D. Thesis, Technical University of Denmark (DTU)). Integration of a transposon into the chromosome of *B. subtilis* may result in the inactivation of a target gene; genes, for which such inactivation led to increased amylase production were sought by screening of transposon libraries for increased production of the chimeric α-amylase AmyLQS55-4 by halo formation on LB-plates containing 0.4 % glucose, 0.01 M phosphate pH 7, 0.2 % xylose, dyed amylopectin, 6 μg/ml Chloramphenicol and 120 μg/ml spectinomycin.

**Example 2:**

**Identification of a gene (SEQ ID NO 1) which when inactivated in a *Bacillus subtilis* cell renders said cell capable of an increased production of an α-amylase:**

A *Bacillus* cell having increased production of the α-amylase was isolated and the identification of a gene (SEQ ID NO 1) was done as described below.
The gene inactivated by the transposon insertion could easily be identified since the mini-Tn*10* contained a pUC origin of replication. In order to rescue DNA flanking the transposon insertion, it was necessary to utilise a restriction enzyme which did not cut within the mini-Tn*10* transposon. Several restriction enzymes could be used for that purpose, however, in this study, the *Eco*RI restriction enzyme was used. In most cases, it was possible to obtain the desired plasmids. In addition to the isolation of the flanking regions surrounding the transposon insertions, the chromosomal DNA was also used for Southern blot analysis, in order to check the number of transposon insertions in each mutant. The Southern blot analysis of the transposon mutants using *Eco*RI restriction enzyme showed that most of the mutants only contained one insertion. Only two of the mutants had more than one transposon inserted in the chromosome.
The flanking regions of the transposon mutants were obtained by digesting the chromosomal DNA with *Eco*RI followed by ligation and transformation by electroporation into electrocompetent cells of *E. coli* SJ2 (Diderichsen, B. et al. 1990, J. Bacteriol. 172:4315-4321), selecting for spectinomycin resistance (120 μg/ml). The rescued plasmids were checked by restriction enzymes analysis and the gene inactivated by the transposon insertion was identified by sequence analysis of the rescued plasmids using primers TKp1 (5'- CCA ATA CGC AAA CGC CCT CTC-3') and TKp2 (5'- TAG TGA CAT TTG CAT GCT TC - 3'). DNA sequences were determined by automatic sequencing. In order to identify the genes which were targeted, the obtained sequences were analysed against the complete genomic sequence of *B. subtilis* (F.Kunst et al., 1997, Nature 390(20): 249-256).
A transposon insertion into the following gene was identified by analysing a mutant obtained as described in Example 1: *yjbH*. The transposon insertion was after nucleotide 711 in the *yjbH* open reading frame.

**Example 3:**

**Confirmation that inactivation of a gene (SEQ ID NO 1) in a *Bacillus subtilis* cell gives improved production of an α-amylase:**

**Accumulation of α-amylase during prolonged growth in very rich media.** The *yjbH* transposon mutant of strain DN1885 *xy1R*::pCJ203, expressing chimeric α-amylase AmyLQS55-4 from a chromosomal gene copy, was grown in shake flasks and the amount of α-amylase accumulated in the culture supernatant compared to the amount accumulated from the non-mutagenised parent strain. After 7 days fermentation at 37°C in BPX medium supplemented with 0.2% xylose and 6 μg/ml chloramphenicol the α-amylase activity in the culture supernatants was measured using the Phadebas assay. A 40% increase in the α-amylase activity was observed for the *yjbH* mutant as compared to the wild-type strain.

**Secretion of α-amylase from *B. subtills* in the stationary growth phase**

The *yjbH* transposon mutant of strain DN1885 xylR::pCJ203, expressing chimeric α-amylase AmyLQS55-4 from a chromosomal gene copy, was also grown in shake flasks containing TY-medium supplemented with 6 μg/ml chloramphenicol and 1% xylose to induce the α-amylase synthesis. During cultivation at 37°C, samples were retrieved to measured the cell density ($OD_{660}$/ml) and the α-amylase activity (NU/ml) in the culture supernatant. The unmutagenised DN1885 xylR::pCJ203 was grown and sampled in parallel. The specific productivity of α-amylase was calculated using the following equation:

$$\texttt{Specific productivity = (dP/dt) x (1/OD}_{\texttt{660}}\texttt{)}$$

Specific productivity (NU/min/$OD_{660}$)
P: production/activity in culture supernatant (NU/ml)
t: growth time (min)
$OD_{660}$: cell density ($OD_{660}$/ml)

The specific activity is a measure of the capacity of the cell to produce α-amylase within a given time period - in this case within one minute. To calculate the specific productivity, equations describing the growth as a function of time and α-amylase activity as a function of time were estimated from the data points obtained during the growth experiment. These two equations were then used to calculate the specific productivity.

An increase in the specific productivity of α-amylase was observed for the *yjbH* mutant as compared to the specific productivity of the parent cell. The specific productivity was approximately 70% higher for this mutant as compared to the parent cell in the post-exponential growth phase.

**Secretion of α-amylase in the exponentially growing *B. subtilis* cells.**

The *Bacillus subtilis* strain DN1885 *xylR*::pCJ199 (Christina Lund Jensen (1997) Secretion of chimeric α-amylases from *Bacillus subtilis*. Ph.D. Thesis, Technical University of Denmark (DTU)) expresses the wild-type α-amylase of *Bacillus licheniformis* from a chromosomally located gene copy. The *yjbH* transposon mutation was transferred to this strain by transformation of competent cells of the recipient with chromosomal DNA prepared from the *yjbH* mutant strain of DN1885 *xylR*::pCJ203. Selection was for spectinomycin resistance.

The DN1885 *xy1R*::pCJ199 strain as well as the yjbH mutant derivative were grown in shake flasks using minimal medium containing 1% ribose as the main carbon source supplemented with 6 μg/ml chloramphenicol and 1% xylose for induction of α-amylase synthesis. During the cultivation at 37°C, samples were retrieved for the measuring of the cell density ($OD_{660}$/ml) and the α-amylase activity in the culture supernatant (NU/ml). The specific productivity of α-amylase was calculated as described above.

The specific productivity was approximately 60 % higher for the *yjbH* mutant as compared to the parent cell during exponential growth.

From these data, it is clear that the *yjbH* mutant is improved with respect to α-amylase production as compared to the wild-type cell.

**Claims**

1. A *Bacillus* cell for improved production of a polypeptide of interest, wherein a gene is inactivated which gene comprises:

    (a) the DNA sequence shown in positions 1 to 828 in SEQ ID NO 1;
    (b) a DNA sequence which is at least 70% identical to the DNA sequence of item (a);
    (c) a DNA sequence which encodes a polypeptide sequence shown in positions 1 to 275 in SEQ ID NO 2; or
    (d) a DNA sequence which encodes a polypeptide sequence which is at least 70% identical to the polypeptide sequence shown in positions 1 to 275 in SEQ ID NO 2.

2. The *Bacillus* cell of claim 1, wherein the gene is situated between the genetic markers *mecA* and *tenA*.

3. The *Bacillus* cell of any of claims 1 to 2, wherein the *Bacillus* cell is a *Bacillus licheniformis, Bacillus subtilis*, or a *Bacillus amyloliquefaciens* cell.

4. The *Bacillus* cell of any of claims 1 to 3, wherein the polypeptide of interest is an enzyme, such as a protease; a cellulase; a lipase; a xylanase; a phospholipase; or preferably an amylase.

**5.** The *Bacillus* cell of any of claims 1 to 4, wherein the gene comprises the DNA sequence shown in positions 1 to 147 in SEQ ID NO 3 or the gene comprises a DNA sequence encoding the polypeptide sequence shown in positions 1 to 49 in SEQ ID NO 4.

**6.** A process for producing a polypeptide of interest comprising following steps:

(i) cultivating a *Bacillus* of any of the preceding claims under conditions permitting production of the polypeptide of interest;
(ii) isolating the polypeptide of interest.

**Patentansprüche**

**1.** *Bacillus*-Zelle zur verbesserten Herstellung eines Polypeptids von Interesse, wobei ein Gen inaktiviert ist, welches Gen umfasst:

(a) die in Positionen 1 bis 828 in SEQ ID NO 1 gezeigte DNA-Sequenz;
(b) eine DNA-Sequenz, die mindestens 70 % identisch zu der DNA-Sequenz von Punkt (a) ist;
(c) eine DNA-Sequenz, die eine in Positionen 1 bis 275 in SEQ ID NO 2 gezeigte Polypeptidsequenz kodiert; oder
(d) eine DNA-Sequenz, die eine Polypeptidsequenz kodiert, die mindestens 70 % identisch zu der in Position 1 bis 275 in SEQ ID NO 2 gezeigten Polypeptidsequenz ist.

**2.** *Bacillus*-Zelle nach Anspruch 1, wobei das Gen zwischen den genetischen Markern *mecA* und *tenA* liegt.

**3.** *Bacillus*-Zelle nach einem beliebigen der Ansprüche 1 bis 2, wobei die *Bacillus*-Zelle eine Zelle von *Bacillus licheniformis, Bacillus subtilis,* oder *Bacillus amyloliquefaciens* ist.

**4.** *Bacillus*-Zelle nach einem der beliebigen der Ansprüche 1 bis 3, wobei das Polypeptid von Interesse ein Enzym, wie eine Protease; eine Cellulase; eine Lipase; eine Xylanase; eine Phospholipase; oder vorzugsweise eine Amylase ist.

**5.** *Bacillus*-Zelle nach einem beliebigen der Ansprüche 1 bis 4, wobei das Gen die in Positionen 1 bis 147 in SEQ ID NO 3 gezeigte DNA-Sequenz umfasst, oder das Gen eine DNA-Sequenz umfasst, kodierend die in Positionen 1 bis 49 in SEQ ID NO 4 gezeigte Polypeptidsequenz.

**6.** Verfahren zum Herstellen eines Polypeptids von Interesse, umfassend die folgenden Schritte:

(i) Kultivieren eines *Bacillus* gemäß einem beliebigen der vorhergehenden Ansprüche unter Bedingungen, die die Herstellung des Polypeptids von Interesse erlauben;
(ii) Isolieren des Polypeptids von Interesse.

**Revendications**

**1.** Cellule de *Bacillus* pour la production améliorée d'un polypeptide d'intérêt, dans laquelle un gène est inactivé, lequel gène comprend:

(a) la séquence d'ADN montrée en positions 1 à 828 dans SEQ ID NO : 1 ;
(b) une séquence d'ADN qui a au moins 70% d'identité avec la séquence d'ADN de (a) ;
(c) une séquence d'ADN qui code une séquence polypeptidique montrée en positions 1 à 275 dans SEQ ID NO : 2 ; ou
(d) une séquence d'ADN qui code une séquence polypeptidique qui a au moins 70% d'identité avec la séquence polypeptidique montrée en positions 1 à 275 dans SEQ ID NO : 2.

**2.** Cellule de *Bacillus* de la revendication 1, dans laquelle le gène est situé entre les marqueurs génétiques *mecA* et *tenA.*

**3.** Cellule de *Bacillus* de la revendication 1 ou 2, ladite cellule de *Bacillus* étant une cellule de *Bacillus licheniformis*, une cellule de *Bacillus subtilis,* ou une cellule de *Bacillus amyloliquefaciens*.

4. Cellule de *Bacillus* de l'une quelconque des revendications 1 à 3, dans laquelle le polypeptide d'intérêt est une enzyme, telle qu'une protéase ; une cellulase ; une lipase ; une xylanase ; une phospholipase ; ou de préférence une amylase.

5. Cellule de *Bacillus* de l'une quelconque des revendications 1 à 4, dans laquelle le gène comprend la séquence d'ADN montrée en positions 1 à 147 dans SEQ ID NO: 3 ou le gène comprend une séquence d'ADN codant la séquence polypeptidique montrée en positions 1 à 49 dans SEQ ID NO : 4.

6. Procédé pour produire un polypeptide d'intérêt comprenant les étapes suivantes :

   (i) la cultivation d'un *Bacillus* de l'une quelconque des revendications précédentes dans des conditions permettant la production du polypeptide d'intérêt ;
   (ii) l'isolement du polypeptide d'intérêt.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0506780 A
- WO 9419454 A

### Non-patent literature cited in the description

- **Kunst et al.** *Nature,* 1997, vol. 390, 249-256
- **Needleman, S.B. ; Wunsch, C.D.** *Journal of Molecular Biology,* 1970, vol. 48, 443-453
- **F.Kunst et al.** *Nature,* 1997, vol. 390 (20), 249-256
- **Fuma, S. et al.** *Nucleic Acids Res.,* 1993, vol. 21 (1), 93-7
- **Tam, N.H. et al.** *Mol Gen Genet.,* 1998, vol. 258 (4), 427-30
- **Lindum et al.** *J Bacteriol.,* 1998, vol. 180 (23), 6384-8
- **Krause, M. et al.** *J Bacteriol.,* 1988, vol. 170 (10), 4669-74
- **Carlson, J.H. et al.** *FEMS Microbiol Lett.,* 1997, vol. 151 (2), 225-30
- **Cole, S.T. et al.** *Nature,* 1998, vol. 393 (6685), 537-44
- **Kong, L. et al.** *Mol Microbiol,* July 1993, vol. 9 (2), 365-73
- **Pang, AS. et al.** *J. Bacteriol,* January 1991, vol. 173 (1), 46-54
- **Guo, D. et al.** *Bio-chim Biophys Acta,* 05 January 1998, vol. 1389 (1), 34-42
- **Ryffel et al.** *Gene,* 28 September 1990, vol. 94 (1), 137-8
- **Tomb, JF. et al.** *Nature,* 07 August 1997, vol. 388 (6642), 539-47
- **Maniatis, T. ; Pritsch, E. F. ; Sambrook, J.** Molecular Cloning. A laboratory manual. Cold Spring Harbor Laboratories, 1982
- Current Protocols in Molecular Biology. John Wiley and Sons, 1995
- Molecular Biological Methods for Bacillus. John Wiley and Sons, 1990
- **Spizizen.** *Proc. Natl. Acad. Sci. USA,* 1958, vol. 44, 1072-1078
- **Steinmetz, M ; Richter, R.** *J. Bacteriol.,* 1994, vol. 172, 5019
- **Diderichsen, B. et al.** *J. Bacteriol.,* 1990, vol. 172, 4315-4321

SEQUENCE LISTING

<110> Novo Nordisk A/S

<120> Gene deletion in Bac -> incr. expression

<130> Gene deletion in Bac -> incr. express

<140>

<141>

<160> 4

<170> PatentIn Ver. 2.1

<210> 1
<211> 828
<212> DNA
<213> Bacillus subtilis

<220>
<221> CDS
<222> (1)..(828)

<400> 1

```
atg ttt gta gac cct tta tgt cct gaa tgc tgg tcc tta gag ccc gtc    48
Met Phe Val Asp Pro Leu Cys Pro Glu Cys Trp Ser Leu Glu Pro Val
1               5                   10                  15

atc aaa aag ctg aaa atc aga tac gga cgt ttt ttc acc tta cgc att    96
Ile Lys Lys Leu Lys Ile Arg Tyr Gly Arg Phe Phe Thr Leu Arg Ile
                20                  25                  30

atc gct tcc gca agc ctt acc gct tta aat aaa aag cga aaa aag cat    144
Ile Ala Ser Ala Ser Leu Thr Ala Leu Asn Lys Lys Arg Lys Lys His
            35                  40                  45

ctt ctc gca gaa gca tgg gaa aag atc gcg agc cgc tct ggc atg tca    192
Leu Leu Ala Glu Ala Trp Glu Lys Ile Ala Ser Arg Ser Gly Met Ser
        50                  55                  60

tgt gac ggc aat gtc tgg ttc gaa cag gat cag ccg ctt tca tcg cct    240
Cys Asp Gly Asn Val Trp Phe Glu Gln Asp Gln Pro Leu Ser Ser Pro
65                  70                  75                  80

tat atg gct gct ctc gct ttt aaa gca gcc gaa ctg caa gga cga aaa    288
Tyr Met Ala Ala Leu Ala Phe Lys Ala Ala Glu Leu Gln Gly Arg Lys
                85                  90                  95

gcc ggc atg caa ttt ctc aga aat atg cag gag agc cta ttt gtt tca    336
Ala Gly Met Gln Phe Leu Arg Asn Met Gln Glu Ser Leu Phe Val Ser
            100                 105                 110

aag aaa aat att acg gat gaa aac gtg ctt ttg gag att gct gaa aat    384
Lys Lys Asn Ile Thr Asp Glu Asn Val Leu Leu Glu Ile Ala Glu Asn
            115                 120                 125

aca agt ctc gat ctt gaa gaa ttc aaa aaa gat ctg cat tct caa agc    432
```

```
        Thr Ser Leu Asp Leu Glu Glu Phe Lys Lys Asp Leu His Ser Gln Ser
            130             135             140

gcg gtc aag gcg ctt caa tgt gac atg aaa att gct gcc gag atg gat        480
Ala Val Lys Ala Leu Gln Cys Asp Met Lys Ile Ala Ala Glu Met Asp
145             150             155             160

gtt tct gtt aat ccg aca ctg acg ttt ttt aat acg cag cat gag gat        528
Val Ser Val Asn Pro Thr Leu Thr Phe Phe Asn Thr Gln His Glu Asp
                165             170             175

gaa ggg ctt aaa gtt cct ggc agc tac tca tat gat gta tat gaa gaa        576
Glu Gly Leu Lys Val Pro Gly Ser Tyr Ser Tyr Asp Val Tyr Glu Glu
            180             185             190

att tta ttt gag atg ctt ggc gac gag ccg aag ccg tcg gaa aca ccg        624
Ile Leu Phe Glu Met Leu Gly Asp Glu Pro Lys Pro Ser Glu Thr Pro
            195             200             205

cct tta gaa tgt ttt att gaa tat ttc cgc ttc gtt gcc tcg aag gaa        672
Pro Leu Glu Cys Phe Ile Glu Tyr Phe Arg Phe Val Ala Ser Lys Glu
            210             215             220

att gct ctt gta tat gac ctg agc ctt gaa gag gta gaa aaa gaa atg        720
Ile Ala Leu Val Tyr Asp Leu Ser Leu Glu Glu Val Glu Lys Glu Met
225             230             235             240

aaa aaa ctg gcg ttt gct aaa aag gtt gcc aaa gtg gaa gcc aaa cac        768
Lys Lys Leu Ala Phe Ala Lys Lys Val Ala Lys Val Glu Ala Lys His
            245             250             255

gga atg ttt tgg aag tct ctc agc act tat tct gat gaa tat caa tca        816
Gly Met Phe Trp Lys Ser Leu Ser Thr Tyr Ser Asp Glu Tyr Gln Ser
            260             265             270

tgt gaa aaa tag                                                        828
Cys Glu Lys
            275
```

<210> 2
<211> 276
<212> PRT
<213> Bacillus subtilis

<400> 2

```
        Met Phe Val Asp Pro Leu Cys Pro Glu Cys Trp Ser Leu Glu Pro Val
            1               5               10              15

        Ile Lys Lys Leu Lys Ile Arg Tyr Gly Arg Phe Phe Thr Leu Arg Ile
                    20              25              30

        Ile Ala Ser Ala Ser Leu Thr Ala Leu Asn Lys Lys Arg Lys Lys His
                    35              40              45

        Leu Leu Ala Glu Ala Trp Glu Lys Ile Ala Ser Arg Ser Gly Met Ser
                50              55              60
```

```
Cys Asp Gly Asn Val Trp Phe Glu Gln Asp Gln Pro Leu Ser Ser Pro
65                  70                  75                  80

Tyr Met Ala Ala Leu Ala Phe Lys Ala Ala Glu Leu Gln Gly Arg Lys
            85                  90                  95

Ala Gly Met Gln Phe Leu Arg Asn Met Gln Glu Ser Leu Phe Val Ser
            100             105             110

Lys Lys Asn Ile Thr Asp Glu Asn Val Leu Leu Glu Ile Ala Glu Asn
            115             120             125

Thr Ser Leu Asp Leu Glu Glu Phe Lys Lys Asp Leu His Ser Gln Ser
    130             135             140

Ala Val Lys Ala Leu Gln Cys Asp Met Lys Ile Ala Ala Glu Met Asp
145             150             155                 160

Val Ser Val Asn Pro Thr Leu Thr Phe Phe Asn Thr Gln His Glu Asp
            165             170             175

Glu Gly Leu Lys Val Pro Gly Ser Tyr Ser Tyr Asp Val Tyr Glu Glu
            180             185             190

Ile Leu Phe Glu Met Leu Gly Asp Glu Pro Lys Pro Ser Glu Thr Pro
        195             200             205

Pro Leu Glu Cys Phe Ile Glu Tyr Phe Arg Phe Val Ala Ser Lys Glu
    210             215             220

Ile Ala Leu Val Tyr Asp Leu Ser Leu Glu Glu Val Glu Lys Glu Met
225             230             235                 240

Lys Lys Leu Ala Phe Ala Lys Lys Val Ala Lys Val Glu Ala Lys His
            245             250                 255

Gly Met Phe Trp Lys Ser Leu Ser Thr Tyr Ser Asp Glu Tyr Gln Ser
            260             265             270

Cys Glu Lys
        275
```

<210> 3
<211> 149
<212> DNA
<213> Bacillus licheniformis

<220>
<221> CDS
<222> (1)..(147)

<400> 3

```
atg ttt gtc gac cct tta tct ccc gaa tgc tgg gca ttg gag ccg gcg    48
Met Phe Val Asp Pro Leu Ser Pro Glu Cys Trp Ala Leu Glu Pro Ala
 1               5                  10                  15

atc aaa aag ttg aaa atc cgc tac ggc cgc ttt ttc aca cta agg atc    96


Ile Lys Lys Leu Lys Ile Arg Tyr Gly Arg Phe Phe Thr Leu Arg Ile
             20                  25                  30

atc gcc gca tgc agc att aca gcg ctg aac gtt cag aaa cgc aaa aag   144
Ile Ala Ala Cys Ser Ile Thr Ala Leu Asn Val Gln Lys Arg Lys Lys
         35                  40                  45

cgc cg                                                            149
Arg
```

<210> 4
<211> 49
<212> PRT
<213> Bacillus licheniformis

<400> 4

```
Met Phe Val Asp Pro Leu Ser Pro Glu Cys Trp Ala Leu Glu Pro Ala
 1               5                  10                  15

Ile Lys Lys Leu Lys Ile Arg Tyr Gly Arg Phe Phe Thr Leu Arg Ile
             20                  25                  30

Ile Ala Ala Cys Ser Ile Thr Ala Leu Asn Val Gln Lys Arg Lys Lys
         35                  40                  45

Arg
```